# EUROPEAN PATENT SPECIFICATION

(11) **EP 3 010 357 B1**
(45) Date of publication and mention of the grant of the patent: **02.01.2019**
(21) Application number: 14813540.3
(22) Date of filing: 13.06.2014
(51) Int. Cl.: A23L 33/21, A23L 33/00, A23L 33/12, A23L 33/17, A61K 9/00, A61K 31/202, A61K 31/201, A61K 31/375, A61K 31/70, A61K 31/714, A61K 38/16, A61K 38/17

(54) **ADMINISTRATION OF A FOOD COMPOSITION PRODUCT**
VERABREICHUNG EINER NAHRUNGSMITTELZUSAMMENSETZUNG
ADMINISTRATION D'UN PRODUIT DE TYPE COMPOSITION ALIMENTAIRE

(30) Priority: 19.06.2013 SE 1350750
(43) Date of publication of application: 27.04.2016
(73) Proprietor: INDEVEX AB (PUBL), 352 46 Växjö (SE)
(72) Inventor: FRANZÉN, Christer, S-598 93 Vimmerby (SE); BUCHAR, Thomas, S-554 71 Jönköping (SE); SEDERHOLM, Magnus, S-116 46 Stockholm (SE)
(74) Representative: Zacco Sweden AB
(86) International application number: PCT/SE2014/050721
(87) International publication number: WO 2014/204382

(56) References cited:
- WO-A1-2005/099483
- WO-A1-2005/099483
- WO-A1-2010/115899
- US-A1- 2002 150 649
- RANIA ABOU-SAMRA ET AL: "Effect of different protein sources on satiation and short-term satiety when consumed as a starter", NUTRITION JOURNAL, BIOMED CENTRAL, GB, vol. 10, no. 1, 23 December 2011 (2011-12-23), page 139, XP021118530, ISSN: 1475-2891, DOI: 10.1186/1475-2891-10-139
- JONATHAN D. BUCKLEY ET AL: "Long-Chain Omega-3 Polyunsaturated Fatty Acids May Be Beneficial for Reducing Obesity-A Review", NUTRIENTS, vol. 2, no. 12, 9 December 2010 (2010-12-09), pages 1212-1230, XP055332258, DOI: 10.3390/nu2121212
- MICHAEL R. LYON ET AL: "Is There a Place for Dietary Fiber Supplements in Weight Management?", CURRENT OBESITY REPORTS, vol. 1, no. 2, 13 April 2012 (2012-04-13), pages 59-67, XP055332576, DOI: 10.1007/s13679-012-0016-9
- CHUN-JUN LI ET AL: "Effects of a Macro-Nutrient Preload on Type 2 Diabetic Patients", FRONTIERS IN ENDOCRINOLOGY, vol. 6, 1 January 2015 (2015-01-01), XP055429743, DOI: 10.3389/fendo.2015.00139

## Description

### TECHNICAL FIELD

The invention relates to a method of administrating of a nutritional composition, the therapeutic diet intervention concept "Preload" including a food composition product "Preload Meal" to be taken before main meals.

### BACKGROUND

65 % of the world's populations live in countries where overweight and obesity kills more people than underweight. Worldwide obesity has nearly doubled during the last three decades, now approx. 1.5 billion adults (20 years and older), were overweight defined as BMI > 25. Of these over 200 million men and 300 million women are obese, defined as BMI > 30. An even more alarming fact is the growing obesity among children; more than 40 million children under the age of five were overweight in 2011. Once considered a high-income country problem, overweight and obesity are now on the rise in low- and middle-income countries, particularly in urban settings.

Similar alarming increasing development of diabetes typ 2 is ongoing in a big number of areas in the world.

Obesity is regarded as major risk factor for several serious diseases such as cardiovascular disorders, hypertension, diabetes typ-2, cancer and osteoarthritis.

Conditions as described above can be prevented through lifestyle modification, diet control, and control of overweight and obesity. Education of the populace is still key to the control of this emerging epidemic. Novel drugs are being developed, yet no definite cure is available in sight for obesity and its alarming consequences like CVD and diabetes type 2. On the other hand life style and diet interventions have scientifically strong support in preventing the onset of e.g. type 2 diabetes as well as to improve the manifest disease.

A common way of dealing with obesity is weight loss through diets. However, diets may lead to poor nutritional intake or even nutritional deficiencies.

There is thus a need for an improved approach towards controlling metabolism and stabilizing blood sugar while still providing essential nutrition in order to fight overweight, obesity and the increased medical risks related to unhealthy diets, diabetes type 2, cardiovascular diseases and cancers. The majority of the popular low calorie diets have failed to show sustainably weight loss. Recently the Diogenes study, supported by the EU Commission, has shown that the only long term sustainable nutrient combination to keep weight stable after weight reduction is combination of high protein content with low GI (N Engl J Med 363;22, 2010)

### SUMMARY OF THE INVENTION

The object of the present invention is to provide a food composition product for use in a method for treating of diabetes type 2. This object is achieved by the features set forth in claim 1. The method is called "Preload" and the food composition product used "Preload Meal", and refers to a load of nutrients 25-35 minutes before a meal in order to reduce postprandial levels of blood sugar.

The invention relates to a food composition product for use in a method for treatment of diabetes type 2. wherein the food composition product is administrated orally to a subject as a 50 to 110 kcal "Preload meal" approximately 25-35 min before administrating a main meal, i.e. breakfast, lunch or dinner, to said subject, wherein said food composition product includes nutrients such that release of incretins in said subject is stimulated prior to said main meal, said food composition product comprising at least three different proteins deriving from both animal and vegetable sources, wherein one source of protein is derived from vellow pea, another source of protein is derived from whey, and a further source of protein is derived from omega 3 rich eggs or egg albumen, wherein the food composition product further comprises Omega 3 and 6 fatty acids and slowly digested carbohydrates including dietary fibers, giving said composition a glycemic load (GL) of <10, thereby increasing satiety of subject and lowering total energy intake including both food composition product and main meal: wherein the carbohydrates comprise foodstuffs from at least apples and sugar beet fibres, and wherein the Preload meal comprises at least 5 and not more than 20 grams of proteins.

WO2005/099483 focuses on preventing/treating metabolic syndrome and provides a food composition product intended to replace an entire meal.
Rania Abou-Samra et al. focus in an article on weight loss and food intake ("Effect of different protein sources on satiation and short-term satiety when consumed as a starter", Nutrition Journal, Biomed Central, GB, vol. 10, no. 1, 23 December 2011 (2011-12-23), page 139, XP021118530, ISSN: 1475-2891, DOI: 10.1186/1475-2891-10-139). The results imply that some proteins may have an effect on the food intake if consumed before the meal.

The method involves the use of a food product composition comprising three proteins sources: pea protein, whey protein, egg albumen, omega 3 rich eggs, apple and sugar root fibre, and orally administrating said food product of said composition to a subject prior to administrating a meal to said subject such that release of incretins in said subject is simulated prior to said meal administration.

The food composition product comprising at least three but even more preferably four proteins sources: leguminous plants preferably pea protein, whey protein, egg albumen and casein protein, furthermore omega 3 and 6 fatty acids, rosaceous plants preferably apple and rose hip, and also fibers preferably from sugar beet root fibre and orally administrating said Preload Meal including said food composition product to a subject prior to administrating a main meal to said subject i.e. breakfast, lunch or dinner, such that release of the gastrointestinal hormones incretins (see below) in said subject is stimulated prior to said main meal administration.

Proteins have multiple points of action in diabetes and weight management; slows the emptying of the stomach contents (takes longer for carbohydrates to be absorbed), stimulate faster release of insulin (rapid uptake of glucose into tissues), stimulates the synthesis of muscle tissue and impact the degree of saturation, etc. through neurotransmitters and neuropeptides.

Pea protein are among the lowest glycemic index (GI) foods and have been recommended in national diabetes mellitus (DM) guidelines. Incorporation of legumes as part of a low-GI diet improves both glycemic control and reduced calculated CHD risk score in type 2 DM in a recent study (Arch Intern Med. 2012 Nov 26; 172(21): 1653-60). It also has been demonstrated that intact pea proteins stimulates CCK and GLP-1 release from human duodenal tissue to a greater extent than egg and codfish protein. Unlike other common protein sources such as milk, soy, or wheat proteins, pea protein has a very low allergenic potential, which makes this protein suitable for dietary interventions (PLoS One. 2011;6(9):e24878). In another study of preload, food intake was significantly lower only after casein and pea protein compared to water control (P = 0.02; 0.04 respectively) (Nutrition Journal 2011, 10:139)

The combination of proteins and omega fatty acids in the food product strengthen the effect. In a recent review of omega fatty acids it was concluded that there is a considerable body of evidence from animal studies indicating that supplementing the diet with LC n-3 PUFA can attenuate weight gain and reduce body fat, in particular epididymal (visceral) fat. Similarly, in human studies there is a growing body of evidence indicating that increasing the intake of LC n-3 PUFA by 0.3-3.0 g/day can reduce body weight and body fat in overweight and obese individuals (Nutrients 2010, 2, 1212-1230). It also has been shown that long-term enteral provision of LCn-3PUFA confers a higher sensitivity to insulin-regulated amino acid and glucose disposal and that these responses probably occur, in part, in skeletal muscle. In a fed steady-state, a more sensitive insulin signalling promotes initiation of protein synthesis with concurrent reduction in whole-body amino acid oxidation, increasing the net availability of amino acids to support anabolism, of paramount importance during weight control (shift from fat body mass to lean body mass).

The inclusion of fibers in the food composition product further promotes satiety at following meals. In a controlled study the fiber supplement significantly reduced hunger feelings and promoted satiety during a period of caloric reduction (Nutr Diabetes. (2011);1:e22). Most clinicians in the field of obesity medicine agree that dietary intervention for long-term weight management should include volumetric, low-calorie-density, low GI diets supplemented with moderate amounts of protein. Increasing the intake of fiber should play a central role in this regard. Supplementation of the diet with functional fibers may significantly promote satiety and reduce cardiometabolic risk factors according to a recent review (Curr Obes Rep (2012) 1:59-67).

The incretins are important gut mediators of insulin release and plasma glucose levels. A food product of the composition administrated prior to a major meal stimulates the release of the incretins GIP and GLP-1. The neuroendocrine response reaches the brain through the systemic route and through afferent neurons. Administration of certain nutrients before a meal, Preload, activates the normal incretin response before the ordinary meal, i.e it has a priming effect on the brain. When the meal is ingested the GIP/GLP-1 already has stimulated the insulin release, promoted satiety and slowed gastric emptying (the priming effect), which in turn reduce meal size and attenuates plasma glucose response.

Preload of proteins/nutrients 25-35 minutes before a meal to the subject is beneficial because the maximum insulin response occurs 30 minutes after ingestion of proteins together with carbohydrates. Thus, initiation of administration of a food product of the composition is to be made 25-35 minutes prior to the administration of a meal.

The Preload Meal composition administered as a preload 25-35 minutes before major meals during a longer period (> 3 months) significantly reduced body weight, BMI and fat%. The food composition product also has positive effects on blood lipids, liver enzymes, insulin levels and HbA1C, and improved life quality parameters on VAS-scales.

Besides proteins nutritional role as a source of "building blocks" to the tissues, dietary proteins affect many vital functions such as glucose and lipid metabolism, blood pressure, bone metabolism and the immune system. Proteins can also function as a "fuel", with approximately the same energy content as carbohydrates. Proteins affect metabolism throughout the entire nutrient uptake, from the oral cavity to the colon. The proteins have several regulatory functions in the gastrointestinal system, including the regulation of food intake. The interaction with signal receptors release hormones that influence gastric emptying and transport of food through the GI system and absorption of nutrients. The proteins also induce nerve signals to the brain (eg, the degree of satiation) through gut receptors and by affecting the intestinal microflora. The interaction between proteins' degradation products (peptides, amino acids) and the endocrine systems also affects digestion.
A dose, i.e. a food product, with a minimum of to 5 grams protein when administrated prior to the administration of a meal is sufficient to affect insulin and glycemic response after the meal.

The four different preferred proteins sources that may be included in a Preload Meal i.e ; yellow pea, chicken egg, whey and casein, have different kinetics and effects on the plasma glucose levels, incretins and satiety system. The ingredient omega 3 fatty acid can potentiate the effect of the proteins regarding the above effects.

The method may also comprise the step of preparing the food product of the composition by mixing the composition with a drinkable liquid. The composition is preferably prepared in dry powder form such that a food product may be prepared as a shake. A serving of the food composition product, prior to a meal, should be approximately 70 kcal. Therefore, the weight of the powder to be mixed in the shake is determined based on calories. The food product may also be prepared as a ready-to-drink liquid comprising a dose of the food composition product.

A food product of the composition for mixing with a drinkable liquid is distinguished primarily by the fact that the composition comprises all the essential food components readily accessible, wherein the relative amounts of the components fat, carbohydrates, proteins, vitamins and minerals of said composition are chosen such that an intake of said composition provides the consumer with a stable blood glucose level.

Said fat, carbohydrates, proteins, vitamins and minerals are derived from a diversity in food groups including fruit, vegetable, plant, dairy, egg and other protein sources, and colloidal water sources, resulting in a physiologically balanced composition.

The "glycemic index" (GI) is a measure of the degree to which the concentration of glucose in the blood rises after consumption of certain foodstuffs. A low glycemic index (GI) here refers to foodstuffs with a GI-value between 0-55. The glycemic index (GI) may be calculated using two different references, that is to say either the reference white bread or the reference glucose. In the measurements made on the food composition product according to the invention, the reference glucose was utilised. In order to estimate the overall glycemic effect of a meal, the concept of "glycemic load" (GL) (GI × dietary carbohydrate content) has been introduced. As the GI compares corresponding amounts of carbohydrates, providing a measure of carbohydrate quantity, but not quality, the GL-value provides the glycemic effect of realistic portion sizes of different foods. The GL-value is similar to the GI-value, a measure of the rise of the blood glucose and the subsequent secretion of insulin in the blood stream, but including the aspect of the amount of carbohydrates available in a portion of food (see Foster-Powell K., Holt SH., Brand-Miller JC., Am. J. Clin. Nutr. 76:5-56, 2002*).*

Such a food composition product formulation is carefully chosen to support the body with essential nutrition. Evidence based medicine support the rationale behind the formula. The food composition product contains a protein, fat and complex carbohydrate combination, based on pure natural sources. It also contains slow carbohydrates and dietary fibers that together with the proteins and fats add up to a low glycemic index of below 40 and more preferably below 30%. With the comparingly low carbohydrate content this adds up into a Io glycemic load (GL) for the Preload Meal af not more than 10 and preferably below 5 which provides a low and stable post prandial blood sugar and equally insulin levels, and in combination with the protein and fat load also a high satiety.

According to one preferred embodiment of the invention, the carbohydrates are derived from leguminous plants, preferably yellow peas, and rosaceous plants, preferably both apples and rose hips, wherein said leguminous plants and rosaceous plants contribute in giving the food composition product its advantageously low Glvalue. However, carbohydrates may, of course within the scope of the invention, be chosen also from other plants having a low glycemic index. Rose hips may furthermore be substituted with pears, peaches, plums, or the like, all of which being rosaceous plants with a low glycemic index. In a further preferred embodiment of the invention, at least 15% of the overall content of the food composition product is derived from yellow peas, at least 10% from apples. The content of the mentioned ingredients preferably does not exceed 30% for yellow peas and 25% for apples or other rosaceous plants so as to maintain the important balance between carbohydrates, proteins and fat.

In addition, said food composition product is also proven to have a satiating effect for a longer period of time after a consumed meal, which effect is of great importance so as to keep feelings of hunger away. As has been disclosed in a previous study (Holt SH., Miller JC., Petocz., Farmakalidis E., Eur. J. Clin. Nutr. 49(9):675-690, 1995), different foods differ greatly in their satiating capacities. The food composition product according to the invention provides said satiating effect due to a number of reasons mentioned in the study above. The low degree of fat in the food composition product is proven advantageous, as fatty foods are shown to be less satisfying, as well as the inclusion of foodstuffs such as eggs and apples, both of which having a high satiety index, as defined in said article. Furthermore, the food composition product according to the invention has a balanced ratio between certain important components such as the contents of protein, fibre, and water. Said components are shown in the above mentioned study to have a positive effect on said satiety.

The food composition product may be balanced to have a GL-value below 10, preferably below 5. As earlier mentioned, carbohydrates are metabolised into glucose by the digestive system of the human body. Until the mid-eighties, it was generally considered that the size of the carbohydrates was of primary importance regarding the blood glucose response. Today, it is known that the same carbohydrate gives rise to different blood glucose responses, due to the form in which it is included in the foodstuffs (see e.g. Björck I., Liljeberg H., Granfeldt Y, Akerberg A., Scand. J. Nutr./Naringsforskning Vol. 40:38-42, 1996). The "glycemic index" (GI) has replaced the terminology of "fast" and "slow" carbohydrates which were related to the size of the sugars included. High GI-values indicate a rapid increase in blood glucose, and low GI-values indicate a delayed absorption rate for the glucose; i.e. carbohydrates with low GI-values are more slowly digested and absorbed. As the level of blood glucose is kept at a more even level, feelings of hunger are kept away for a prolonged period also avoiding unnecessary and unhealthy cravings for foodstuffs rich in carbohydrates. Thus, said problems are avoided with the food composition product having said GL-value, which GL-value is a product of a low GI-value and the amount of available carbohydrates. However, in addition to the food composition product having a low GL-value, the satiating effect of said product is of equal importance in order to avoid feelings of hunger.

However, even though carbohydrates from different sources may be associated with individual GI-values, the GI-value of each of the carbohydrates put together does not determine the GI of the foodstuffs. The GI is influenced by a number of factors, for example, the biochemical structure of the carbohydrate, a high amylose/amylopectin ratio, a high degree of native starch, presence of anti-nutritional substances with the ability of inhibiting amylose, and the co-ingestion of fat, fibre and protein. A problem thus occurs when trying to compose a meal which is to provide the consumer with a low glycemic index for the overall food composition product. Due to the contribution of a number of factors to the glycemic index, a meal properly balanced so as to avoid fluctuations in the blood glucose level may be difficult to compose.

Furthermore, the choice of carbohydrate source is, as also mentioned, another important factor in obtaining a low GL food composition product. Accordingly, carbohydrates having low GI-values are chosen. According to one embodiment of the invention the food composition product has a content comprising yellow peas, apples, and rose hips as described above, all of which comprise carbohydrates with low GI-values. The present inventors have found that when foodstuffs with an average GI-value, that is between 60-90, are included, the overall food composition product obtains a significantly higher GL-value when compared to a food composition product comprising only carbohydrates with a low GI-value.

The carbohydrates, when decomposed provide the human body system with simple sugars from the group consisting of glucose, saccharose, fructose, maltose and lactose.

However, it is also possible to alter the contents of the different types of sugar within the scope of the invention. In one embodiment according to the invention the food composition product is low lactose or completely free from lactose in order to meet the demands for such products.

According to one embodiment of the invention, the food composition product comprises physiological doses of dietary fibres, i.e. between 3-15g of 100g respectively 1,5 - 7,5g of 100kcal of a Prelaod Meal preferably about 5-10g per 100g resp 2,5-5g per 100kcal and most preferably 6g per serving in a non liquid Preload Meal. The GL of a meal is affected by the content of fibres, as a high degree of fibres helps to lower the glycemic index. Fibres are not digested, but are however necessary in order for the bowels to function correctly. The content of fibres in the food composition product according to the invention is advantageous in improving motility (bowel movements) and thus enhancing the consumer's ability to process food efficiently. In addition, the fibres help the elimination of toxins in the cells and furthermore is recognized to reduce the cholesterol content in the blood system.

In a preferred embodiment according to the invention the fat of the Preload Meal comprises the essential fatty acids with an omega-3 to omega-6 fatty acid ratio of 1:0.8 to 1:4.0, more preferably 1:1.5 to 1:2.5, and most preferably 1:2. A correct balance is significant in order to maintain normal cellular and other functions. As a typical western diet consists of far more omega-6 fatty acids than omega-3 fatty acids, many meals are lacking the essential amount of the desired fatty acids and have moreover an inadequate balance of said fatty acids. The fat of the composition is further composed to meet the needs for saturated, and mono-, di-, tri- and polyunsaturated fatty acids. The fat preferably comprises preferably 20-40% saturated fatty acids, preferably 20-40% monounsaturated fatty acids, and preferably 20-40% di- and tri-unsaturated fatty acids. The balance between the three main groups of fatty acids is thus optimised. The content of fat in a properly balanced meal is essential; fats contain important fat soluble vitamins and essential fatty acids which cannot be produced by the human body. The fatty acids are preferably chosen from the group consisting of myristic acid, palmitic acid, palmitoleic acid, heptadecanoic acid, stearic acid, oleic acid, linoleic acid, alfa-linolenic acid, arachidic acid, eicosadienoic acid, behenic acid, docosahexaenoic acid (DHA) and eicosapentaenoic acid (EPA). However, the invention is not restricted to the fatty acids mentioned herein. Other fatty acids which are within the scope of the invention may also be chosen. In addition, the levels of docosahexaenoic acid (DHA) and eicosapentaenoic acid (EPA) are optimised and constitute between 1.9-2.3%, preferably about 2.1% of the total fat. Cholesterol is also present in amounts which meet the body's needs.

The composition is furthermore an excellent source of protein, thus containing all 20 amino acids including the 8 for adult by WHO recognized essential amino acids in physiological doses. The composition further comprise vitamins comparingly high amounts of vitamin B12 and C.

The food composition product mixed with drinkable liquid preferably has a pH-range of 5.5 to 6.5 and acts like a buffer.

In order to meet the demands of people with gluten intolerance, the food composition product, Preload Meal, is preferably gluten free.

Furthermore, said food composition product may also be provided in the form of a bar, thus a Preload Meal food product of the composition is easy to administrate and consume. The bar may be produced by any method known in the art.

The nutritive substances of the food composition product according to the invention are derived from both animal and vegetable sources, thereby providing all the amino acids, including the essential amino acids, fatty acids, and slow-working carbohydrates in a proper balance, all of which contribute to a low glycemic index. No artificial sweeteners, preservatives or thickening agents are added to the food composition product.

All ingredients are mechanically treated (heat, air, steam, pressure) to form a powder and are free from chemicals and are GMO free.

Said food composition product has also proved to provide a stable blood glucose level, to prevent insulin spikes. In addition, basal insulin levels are lowered in all people, and long acting (basal insulin level) and short acting (insulin spike) insulin requirements are significantly reduced with insulin dependent diabetics.

In an embodiment of the invention the food composition product the components includes a protein content of more than 20% calculated from the food composition products energy content; and thus according to European/EFSA regulations classified as a "protein rich" food composition.

### DETAILED DESCRIPTION OF THE INVENTION

### Study on the effect of administrating the composition prior to a meal

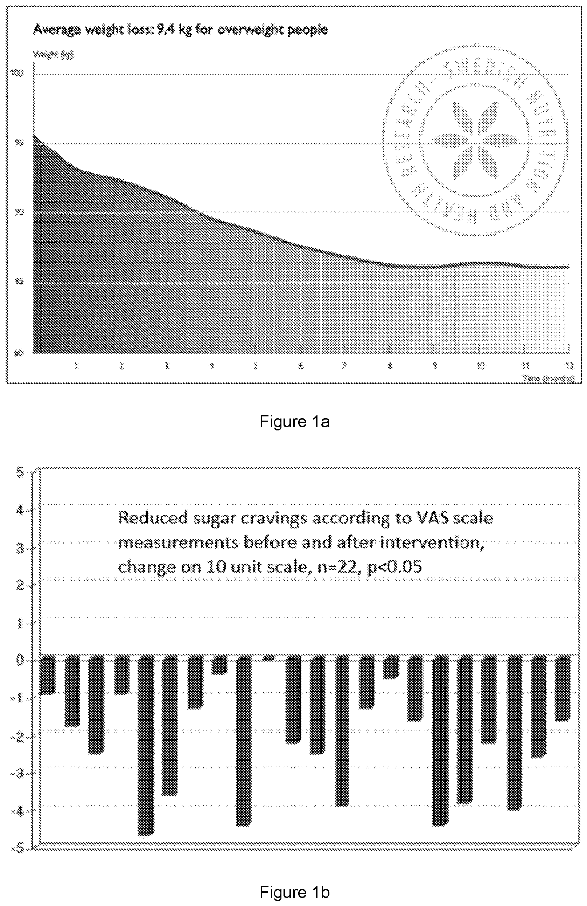

In a study performed at Lund University a cohort of obese subjects was administrated a dose of the composition, as a water soluble dry powder/shake, 2 to 3 times a day as preload before major meals. Parameters linked to obesity and diabetes type 2 was documented. 12 subjects with a BMI > 30, 8 women and 4 men with a mean age of 43 years, ranging between 25 and 58 years, participated in a special program for weight reduction. The program was constituted by intake of the food composition product and access to a nutritionist by whom the participants was given nutritional advice as well as guidance to a healthier life style. The included subjects were non-smokers and, besides the overweight, without any known diseases. Medical examination was performed at the start of the program and at 3, 6, 12 and 24 months for blood sampling, answering questions, and to get advice. DEXA, blood pressure, spirometry, ECG, body weight, height, circumference of waist and hip were recorded.
The mean body weight at the end of each period of the 1 year and 2 year groups decreased with 9.4 kg and 13.6 kg, respectively compared to baseline. Figure 1a shows the average weight loss over the first 12 months. The BMI index decreased in analogy with the decrease in body weight. The waist and bottom circumferences also showed substantial decreases parallel to the body weight and BMI-index curves. In the VAS scale measurements, the desire for sweet and candies markedly reduced in the 1 and 2 year groups in all subjects. The level of energy and amount of exercise increased markedly for all subjects in both groups. All subjects in the 2 year group reported an increased feeling of general well-being.

In conclusion; the food composition product administered as a Preload Meal 30 minutes before major meals significantly reduced body weight, BMI and fat%. The composition also had positive effects on blood lipids, liver enzymes, insulin levels and HbA1C, and improved life quality parameters on VAS-scales.

As seen in Figure 1a the subjects participating in the program had a substantial weight loss during the first year. During the second year the weight loss continued (2 year group). The BMI- values decreased from 33.4 to 30.5 in the 1 year group and from 34.9 to 30.3 in the 2 year group which is close to a WHO classification as overweight instead of being classified as obese. The circumference of waist and hip decreased in analogy with the weight. In parallel there was a reduction of the subjects' blood pressure.

The first three months after inclusion in the program the insulin levels reduced significantly and then levelled out at values between 9 and 10 mU/L. As long as a subject had a high insulin level it was impossible for him/her to lose weight, but when the insulin was reduced he/she started to lose weight. HbA1c reduced significantly the first three months and then there was an increase so that the values leveled out around 4.4%, which is normal.

Figure 1b shows the reduced sugar cravings according to VAS scale measurements before and after internention, change on 10 unit scale, n=22, p<0.05.

## Claims

1. A food composition product for use in a method for treatment of diabetes type 2, wherein the food composition product is administrated orally to a subject as a 50 to 110 kcal "Preload Meal" approximately 25-35 min before administrating a main meal, i.e. breakfast, lunch or dinner, to said subject, wherein said food composition product includes nutrients such that release of incretins in said subject is stimulated prior to said main meal, said food composition product comprising at least three different proteins deriving from both animal and vegetable sources, wherein one source of protein is derived from yellow pea, another source of protein is derived from whey, and a further source of protein is derived from omega 3 rich eggs or egg albumen, wherein the food composition product further comprises omega 3 and 6 fatty acids and slowly digested carbohydrates including dietary fibers, giving said composition a glycemic load (GL) of <10, thereby increasing satiety of subject and lowering total energy intake including both food composition product and main meal; wherein the carbohydrates comprise foodstuffs from at least apples and sugar beet fibres, and wherein the Preload Meal comprises at least 5 and not more than 20 grams of proteins.

2. A food composition product for use according to claim 1, wherein the method comprises the step of preparing the Preload Meal by mixing the nutrients with a drinkable liquid.

3. A food composition product for use according to any of the preceding claims, wherein at least 15% of the overall content of the food composition product is derived from yellow peas, at least 10% from apples.

4. A food composition product for use according to any of the preceding claims, wherein the food composition product comprises dietary fibres providing 3-15g of 100g respectively 1.5-7.5g of 100kcal of a Preload Meal, preferably about 5-10g per 100g resp 2.5-5g per 100kcal and most preferably 6g per 100g resp 3g per 100kcal in a Preload Meal.

5. A food composition product for use according to any of the preceding claims, wherein the fat comprises the essential fatty acids with an omega-3 to omega-6 fatty acid ratio of 1:0.8 to 1:4, more preferably 1:1.5 to 1:2.5, and most preferably 1:2.

6. A food composition product for use according to any of the preceding claims, wherein the fatty acids comprise 20-40%, saturated fatty acids, 20-40%, monounsaturated fatty acids, and 20-40%, di- and tri-unsaturated fatty acids.

7. A food composition product for use according to any of the preceding claims, wherein the food composition product comprises all 20 amino acids including the 8 essential amino acids.

8. A food composition product for use according to any of the preceding claims, wherein the food composition product comprises vitamins B, C, which are present in high amounts, and sodium in small amounts, in physiological doses.

9. A food composition product for use according to any of the preceding claims, wherein the food composition product is in the pH-range of 5.5 to 6.5 and acts like a buffer.

10. A food composition product for use according to any of the preceding claims, wherein the food composition product comprises no artificial preservatives or thickening agents.

## Patentansprüche

1. Nahrungsmittelzusammensetzungsprodukt zur Verwendung in einem Verfahren zur Behandlung von Typ-2-Diabetes, wobei das Nahrungsmittelzusammensetzungsprodukt als eine 50 bis 110 kcal-enthaltende "Vormahlzeit" ca. 25-35 Minuten vor Verabreichung einer Hauptmahlzeit, d.h. Frühstück, Mittagessen oder Abendbrot, an ein Individuum oral verabreicht wird, wobei das Nahrungsmittelzusammensetzungsprodukt Nährstoffe enthält, so dass Freigabe von Inkretinen im Individuum vor der Hauptmahlzeit angeregt wird, wobei das Nahrungsmittelzusammensetzungsprodukt mindestens drei verschiedene Proteine stammend aus sowohl animalischen als auch pflanzlichen Quellen umfasst, wobei eine Proteinquelle aus Rankenplatterbse stammt, eine andere Proteinquelle aus Molke stammt, und eine weitere Proteinquelle aus Omega-3-reichen Eiern oder Eiweiß stammt, wobei das Nahrungsmittelzusammensetzungsprodukt weiter Omega-3 und -6-Fettsäuren und langsam verdauliche Kohlenhydrate einschließlich Ballaststoffe umfasst, wodurch die Zusammensetzung eine glykämische Last (GL) von <10 bekommt, wodurch die Sattheit des Individuums erhöht wird und die gesamte Energieaufnahme einschließlich sowohl Nahrungsmittelzusammensetzungsprodukt als auch Hauptmahlzeit vermindert wird; wobei die Kohlenhydrate Nährmittel aus mindestens Äpfeln und Zuckerrübenballaststoffen umfassen, und wobei die Vormahlzeit mindestens 5 und höchstens 20 Gramm Protein umfasst.

2. Nahrungsmittelzusammensetzungsprodukt zur Verwendung nach Anspruch 1, wobei das Verfahren den Schritt zur Herstellung der Vormahlzeit durch Mischen der Nährstoffe mit einer trinkbaren Flüssigkeit umfasst.

3. Nahrungsmittelzusammensetzungsprodukt zur Verwendung nach einem der vorgehenden Ansprüche, wobei mindestens 15% des gesamten Gehalts des Nahrungsmittelzusammensetzungsprodukts aus Rankenplatterbsen und mindestens 10% aus Äpfeln stammt.

4. Nahrungsmittelzusammensetzungsprodukt zur Verwendung nach einem der vorgehenden Ansprüche, wobei das Nahrungsmittelzusammensetzungsprodukt Ballaststoffe umfasst, wodurch sich 3-15 g von 100 g bzw. 1,5-7,5 g von 100 kcal einer Vormahlzeit, vorzugsweise ca. 5-10 g pro 100 g bzw. 2,5-5 g pro 100 kcal und am meisten bevorzugt 6 g pro 100 g bzw. 3 g pro 100 kcal in einer Vormahlzeit ergibt.

5. Nahrungsmittelzusammensetzungsprodukt zur Verwendung nach einem der vorgehenden Ansprüche, wobei das Fett die essentiellen Fettsäuren mit einem Omega-3 zu Omega-6-Fettsäurenverhältnis von 1:0,8 bis 1:4, vorzugsweise 1:1,5 bis 1:2,5 und am meisten bevorzugt 1:2 umfasst.

6. Nahrungsmittelzusammensetzungsprodukt zur Verwendung nach einem der vorgehenden Ansprüche, wobei die Fettsäuren 20-40% gesättigte Fettsäuren, 20-40% monoungesättigte Fettsäuren und 20-40% di- und trigesättigte Fettsäuren umfassen.

7. Nahrungsmittelzusammensetzungsprodukt zur Verwendung nach einem der vorgehenden Ansprüche, wobei das Nahrungsmittelzusammensetzungsprodukt alle 20 Aminosäuren einschließlich der 8 essentiellen Aminosäuren umfasst.

8. Nahrungsmittelzusammensetzungsprodukt zur Verwendung nach einem der vorgehenden Ansprüche, wobei das Nahrungsmittelzusammensetzungsprodukt Vitamin B, C, die in großen Mengen vorkommen, und Natrium in kleinen Mengen, in physiologischen Dosen umfasst.

9. Nahrungsmittelzusammensetzungsprodukt zur Verwendung nach einem der vorgehenden Ansprüche, wobei das Nahrungsmittelzusammensetzungsprodukt im pH-Bereich von 5,5 bis 6,5 liegt und wie ein Puffer funktioniert.

10. Nahrungsmittelzusammensetzungsprodukt zur Verwendung nach einem der vorgehenden Ansprüche, wobei das Nahrungsmittelzusammensetzungsprodukt keine künstlichen Konservierungsmittel oder Verdickungsmittel umfasst.

## Revendications

1. Produit de composition alimentaire pour l'usage dans un procédé de traitement du diabète de type 2, dans lequel le produit de composition alimentaire est administrée par voie orale à un sujet en tant que "Repas de précharge" avec 50 à 110 kcal environ 25 à 35 minutes avant l'administration d'un repas principal, à savoir le petit déjeuner, le déjeuner ou le dîner, audit sujet, dans lequel ledit produit de composition alimentaire inclut des éléments nutritifs si bien que la libération d'incretins chez ledit sujet est stimulée avant ledit repas principal, ledit produit de composition alimentaire comprenant au moins trois protéines différentes provenant à la fois de sources animales et végétales, dans lequel une source de protéine provient de pois jaune, une autre source de protéine provient de lactosérum, et une source supplémentaire de protéine provient d'oeufs riches en oméga 3 ou du blanc de l'oeuf, dans lequel ledit produit de composition alimentaire comprend en outre des acides gras d'oméga 3 et 6 ainsi que des carbohydrates à digestion lente comprenant des fibres alimentaires, conférant à cette composition un charge glycémique (GL) de <10, augmentant ainsi la satiété du sujet et réduisant l'apport énergétique total, y compris le produit de composition alimentaire et le repas principal ; dans lequel les carbohydrates comprennent des produits alimentaires provenant au moins de pommes et de fibres de betteraves, et dans lequel le repas de précharge comprend au moins 5 et pas plus que 20 grammes de protéines.

2. Produit de composition alimentaire pour l'usage selon la revendication 1, dans lequel le procédé comprend l'étape consistant à préparer le repas de précharge en mélangeant les nutriments avec un liquide buvable.

3. Produit de composition alimentaire pour l'usage selon l'une quelconque des revendications précédentes, dans lequel au moins 15% de la teneur totale du produit de composition alimentaire provient de pois jaunes, au moins 10% de pommes.

4. Produit de composition alimentaire pour l'usage selon l'une quelconque des revendications précédentes, dans lequel le produit de composition alimentaire comprend des fibres alimentaires fournissant 3-15g de 100g respectivement 1,5-7,5g de 100kcal d'un repas de précharge, de préférence d'environ 5-10g pour 100g respectivement 2,5-5g pour 100kcal et le plus préférablement 6g pour 100g respectivement 3g pour 100kcal dans un repas de précharge.

5. Produit de composition alimentaire pour l'usage selon l'une quelconque des revendications précédentes, dans lequel la graisse comprend les acides gras essentiels avec un rapport acides gras oméga-3 à oméga-6 de 1:0,8 à 1:4, plus préférablement 1:1,5 à 1:2,5, et le plus préférablement 1:2.

6. Produit de composition alimentaire pour l'usage selon l'une quelconque des revendications précédentes, dans lequel les acides gras comprennent 20 à 40% d'acides gras saturés, 20 à 40% d'acides gras mono-insaturés et 20 à 40% d'acides gras di et tri-insaturés.

7. Produit de composition alimentaire pour l'usage selon l'une quelconque des revendications précédentes, dans lequel le produit de composition alimentaire comprend la totalité des 20 acides aminés, y compris les 8 acides aminés essentiels.

8. Produit de composition alimentaire pour l'usage selon l'une quelconque des revendications précédentes, dans lequel le produit de composition alimentaire comprend des vitamines B, C, qui sont présentes en grande quantité, et du sodium en petite quantité, à des doses physiologiques.

9. Produit de composition alimentaire pour l'usage selon l'une quelconque des revendications précédentes, dans lequel le produit de composition alimentaire est dans la gamme de pH de 5,5 à 6,5 et agit comme un tampon.

10. Produit de composition alimentaire pour l'usage selon l'une quelconque des revendications précédentes, dans lequel le produit de composition alimentaire ne comprend aucun agent de conservation artificiel ni agent épaississant
